# EUROPEAN PATENT APPLICATION

(11) **EP 2 762 189 A1**
(43) Date of publication of application: **06.08.2014**
(21) Application number: 14152817.4
(22) Date of filing: 28.01.2014
(51) Int. Cl.: A61M 25/00, A61M 25/09

(54) **Slitted pipe and guide wire using the same**

(30) Priority: 31.01.2013 JP 2013016964; 31.01.2013 JP 2013016965; 30.09.2013 JP 2013202969
(71) Applicant: ASAHI INTECC CO., LTD., Nagoya-shi, Aichi 463-0024 (JP)
(72) Inventor: Kimura, Kazuyuki, Nagoya-shi, Aichi 463-0024 (JP)
(74) Representative: Winter, Brandl, Fürniss, Hübner, Röss, Kaiser, Polte - Partnerschaft

(57) **Abstract**

A slitted pipe (10) includes a first slit pattern (15) helically extending in a longitudinal direction of the slitted pipe (10) and divided by girders (13) into a plurality of slits at predetermined intervals, and a second slit pattern (16) extending in the longitudinal direction to be helical in a direction opposite from a helical direction of the first slit pattern (15), and disposed at an angle to the first slit pattern (15) to cross the girders (13).

## Description

### BACKGROUND OF THE INVENTION

### 1. Field of the Invention

The present invention relates to a slitted pipe for use in a medical instrument to be inserted in a body cavity for medical treatment and inspection, and a guide wire using the slitted pipe.

### 2. Description of the Related Art

There have hitherto been proposed various medical instruments to be inserted in a tubular organ such as a blood vessel, a digestive tract, or a ureter, or a body tissue for medical treatment and inspection.

For example, Japanese Unexamined Patent Application Publication (Translation of PCT Application) No. 2009-519103 (Patent Document 1) describes a medical instrument including a hypotube in which a plurality of cuts, such as grooves, are helically formed in a tubular member. Some of the grooves provided in the tubular member are each composed of a first part extending at an acute angle to an axis of the tubular member and a second part connected substantially orthogonally to the first part.

### SUMMARY OF THE INVENTION

For example, in a case in which a medical instrument, such as a guide wire, enters a chronic total occlusion lesion, even when an operator pushes in the guide wire deep while rotating a proximal end of the guide wire in both clockwise and counterclockwise directions, a distal end of the guide wire does sometimes not sufficiently advance into the chronic total occlusion lesion. It is considered that this is because transmissibility of push-in torque for properly advancing the distal end of the guide wire deep is not sufficiently exerted when the guide wire is pushed in in an axial direction.

In the medical instrument described in Patent Document 1, it is difficult to ensure sufficient transmissibility of push-in torque. In this respect, there is still room for improvement in the medical instrument.

The present invention has been made in view of these circumstances, and an object of the invention is to provide a slitted pipe that can easily ensure sufficient transmissibility of push-in torque in both clockwise and counterclockwise rotations, and a guide wire using the slitted pipe.

To achieve the above object, a slitted pipe and a guide wire using the slitted pipe according to aspects of the present invention have the following characteristics.

A slitted pipe according to a first aspect of the present invention includes at least one first slit pattern helically extending in a longitudinal direction of the slitted pipe and divided by girders at predetermined intervals, and at least one second slit pattern helically extending in the longitudinal direction and disposed at an angle to the first slit pattern to cross the girders.

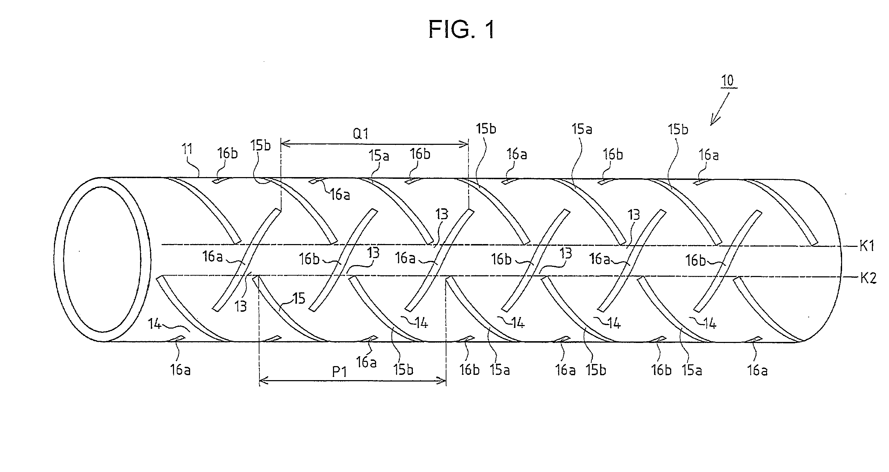

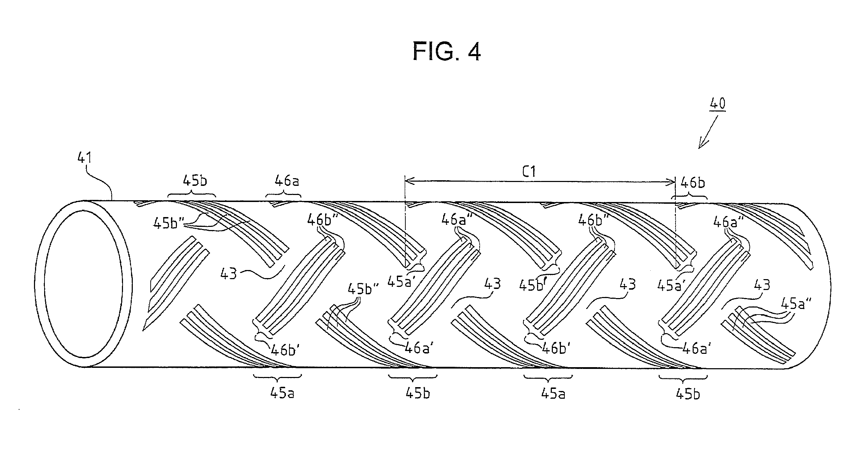

Here, "a slit pattern" means a helical pattern comprising a plurality of slits (Fig.1 to 3) divided by the girders and arranged on the same helical shape in a row. Alternatively, "a slit pattern" may also comprise a plurality of slit groups (Fig.4) divided by the girders and arranged on the same helical shape in a row. A slit group comprises a plurality of slits disposed adjacent and parallel to one another (Fig.4).

While the slitted pipe of the present invention comprises at least one first slit pattern and at least one second slit pattern, the slitted pipe of the present invention may also comprise a plurality of parallel first slit patterns and a plurality of parallel second slit patterns (Fig. 1 to 4).

In the slitted pipe of the first aspect, both a Z-helical (right-handed helical) slit pattern and an S-helical (left-handed helical) slit pattern are provided apart from each other.

As a result, for example, when the slitted pipe is pushed into a blood vessel while being rotated in the clockwise direction, it is compressed to decrease the distances between adjacent slits of the right-handed helical slit pattern, and stress produced at this time acts in a push-in direction. Hence, transmissibility of push-in torque is enhanced, and this allows a distal end portion of the slitted pipe to properly advance deep into the blood vessel.

In contrast, when the slitted pipe is pushed into the blood vessel while being rotated in the counterclockwise direction, it is similarly compressed to decrease the distances between adjacent slits of the left-handed helical slit pattern, and stress produced at this time acts in the push-in direction. Hence, transmissibility of push-in torque is enhanced, and this allows the distal end portion of the slitted pipe to properly advance deep into the blood vessel.

That is, when the slitted pipe is pushed into the blood vessel while being rotated in both the clockwise and counterclockwise directions, transmissibility of the push-in torque that is equivalently high in both the clockwise and counterclockwise directions is obtained. Therefore, for example, the slitted pipe easily advances deep into a chronic total occlusion lesion, and operability is enhanced.

A guide wire according to a second aspect of the present invention includes a core shaft, and the above slitted pipe covering a distal end portion of the core shaft.

According to the guide wire of the second aspect, it is possible to obtain a guide wire that easily ensures sufficient transmissibility of push-in torque and that can properly advance deep into a blood vessel.

A guide wire according to a third aspect of the present invention includes a core shaft, a coil body covering a distal end portion of the core shaft, and the above slitted pipe disposed in the coil body to cover the distal end portion of the core shaft.

According to the guide wire of the third aspect, it is possible to obtain a guide wire that easily ensures sufficient transmissibility of push-in torque and that can properly advance deep into a blood vessel. Also, it is possible to ensure high flexibility of the guide wire by the slitted pipe disposed in the coil body while the outer diameter of the coil body is maintained.

### BRIEF DESCRIPTION OF THE DRAWINGS

Fig. 1 is a perspective view of a slitted pipe according to a first embodiment of the present invention;
Fig. 2 is a perspective view of a slitted pipe according to a second embodiment of the present invention;
Fig. 3 is a perspective view of a slitted pipe according to a third embodiment of the present invention;
Fig. 4 is a perspective view of a slitted pipe according to a fourth embodiment of the present invention;
Fig. 5 is an overall view of a first embodiment of a guide wire according to the present invention; and
Fig. 6 is an overall view of a second embodiment of a guide wire according to the present invention.

### DESCRIPTION OF THE PREFERRED EMBODIMENTS

First, slitted pipes according to preferred embodiments of the present invention will be described with reference to the drawings.

### First Embodiment

Fig. 1 is a perspective view of a slitted pipe according to a first embodiment of the present invention. In Fig. 1 and Figs. 2 to 4 described below, for easy understanding, the slitted pipe is schematically illustrated as a whole while being shortened in a longitudinal direction. Hence, overall dimensions of the slitted pipe are different from actual ones.

As illustrated in Fig. 1, a slitted pipe 10 of the first embodiment includes two parallel first slit patterns 15a, 15b that helically extend in a longitudinal direction of a tubular member 11. The first slit patterns 15a and 15b are adjacent to each other in the longitudinal direction of the tubular member 11, have the same structure, and run parallel to each other. The first slit patterns 15a and 15b are shifted from each other by 180° in the circumferential direction of the slitted pipe 10. The first slit patterns 15a, 15b each are divided into a plurality of slits by girders 13 at predetermined intervals. In the first embodiment, the first slit patterns 15a, 15b form a right-handed helical shape in the longitudinal direction of the tubular member 11.

The slitted pipe 10 further includes two parallel second slit patterns 16a, 16b. The second slit patterns 16a and 16b are adjacent to each other in the longitudinal direction of the tubular member 11, have the same structure, and run parallel to each other. The second slit patterns 16a and 16b are shifted from each other by 180° in the circumferential direction of the slitted pipe 10. The second slit patterns 16a, 16b extend in the longitudinal direction of the tubular member 11 to be helical in a direction opposite from the first slit patterns 15a, 15b (to form a left-handed helical shape in the longitudinal direction of the tubular member 11), and cross the above-described girders 13. That is, the slits of the second slit patterns 16a, 16b are not in contact with the slits of the first slit patterns 15a, 15b, and the second slit patterns 16a, 16b are disposed at an angle to the first slit patterns 15a, 15b.

Hence, in the slitted pipe 10, the first slit patterns 15a, 15b extending in the right-handed helical shape in the longitudinal direction of the tubular member 11 and the second slit patterns 16a, 16b extending in the left-handed helical shape in the longitudinal direction of the tubular member 11 are provided apart from each other.

As a result, for example, when a below-described guide wire including the slitted pipe 10 is pushed into a blood vessel while a proximal end portion thereof is being rotated clockwise, it is compressed to reduce distances between adjacent slits of the right-handed helical first slit patterns 15a, 15b, and stress produced at this time acts in a push-in direction. Hence, transmissibility of push-in torque is enhanced, and a distal end portion of the guide wire can properly advance deep into the blood vessel.

In contrast, when the guide wire is pushed into the blood vessel while the proximal end portion thereof is being rotated counterclockwise, it is similarly compressed to reduce distances between adjacent slits of the left-handed helical second slit patterns 16a, 16b, and stress produced at this time acts in the push-in direction. Hence, transmissibility of push-in torque is enhanced, and the distal end portion of the guide wire can properly advance deep into the blood vessel.

That is, when the distal end portion of the guide wire is pushed into the blood vessel while the proximal end portion of the guide wire is being rotated in both the clockwise and counterclockwise directions, it is possible to obtain push-in torque transmissibility that is equivalently excellent in both the clockwise and counterclockwise directions. Therefore, the guide wire can easily advance into, for example, a chronic total occlusion lesion, and operability is enhanced.

In the first embodiment, as illustrated in Fig. 1, the first slit patterns 15a, 15b and the second slit patterns 16a, 16b are arranged line-symmetrically to each other with respect to an axis. Hence, both the first slit patterns 15a, 15b and the second slit patterns 16a, 16b are disposed symmetrically and uniformly.

As a result, for example, in the below-described guide wire including the slitted pipe 10, torque transmissibility can be substantially equivalent when the guide wire is rotated clockwise and when the guide wire is rotated counterclockwise, and torque transmissibility well balanced in a circumferential cross section can be ensured easily. Therefore, it is possible to enhance operability of the guide wire when the guide wire is inserted deep into the blood vessel.

In the first embodiment, as illustrated in Fig. 1, the slits of the first slit pattern 15a, 15b and the slits of the second slit patterns 16a, 16b are substantially equal in width.

In the first embodiment, a pitch P1 of the first slit pattern 15a is substantially fixed. In addition, a pitch Q1 of the second slit pattern 16a is substantially fixed. The pitch P1 of the first slit pattern 15a and the pitch Q1 of the second slit pattern 16a are substantially equal to each other. Hence, torque transmissibility of the slitted pipe 10 itself can be enhanced in a well-balanced manner in the circumferential cross section.

While a method for forming the first slit patterns 15a, 15b and the second slit patterns 16a, 16b is not particularly limited, for example, laser machining or etching is used.

Since the slitted pipe 10 has the first and second slit patterns in its surface, flexibility thereof can be enhanced. Hence, when the slitted pipe 10 is inserted in a blood vessel of a patient, perforation of the blood vessel can be prevented effectively.

Since the slitted pipe 10 has a plurality of helical slit patterns in its surface, play is formed when rotational force applied to one end of the slitted pipe 10 is transmitted to the other end. Hence, even if great force is suddenly applied to one end of the slitted pipe 10, it can be prevented from being immediately transmitted to the other end. Thus, an accident can be avoided.

As illustrated in Fig. 1, in the first embodiment, the girders 13 that divide the first slit patterns 15a, 15b are linearly linked along the axis of the slitted pipe 10 from one end toward the other end. That is, the girders 13 are located in an area surrounded by a pair of parallel virtual lines K1 and K2. Similarly, girders 14 that divide the second slit patterns 16a, 16b into plurality of slits 16a', 16b' are linearly linked along the axis from one end toward the other end.

Hence, expansion and contraction of the slitted pipe 10 in the axial direction are prevented, and transmissibility of push-in torque of the slitted pipe 10 can be enhanced further.

For example, the cross section of the tubular member 11 that forms the slitted pipe 10 of the first embodiment is shaped like a substantially perfect circle, an ellipse, or a polygon such as a triangle, a rectangle, a hexagon, or an octagon. Among of these, in the first embodiment, the cross-section of the tubular member 11 is preferably shaped like a substantially perfect circle from a viewpoint of uniform transmission of rotational torque over the entire circumference.

While the first slit patterns 15a, 15b and the second slit patterns 16a, 16b are arranged line-symmetrically with respect to the axis in the first embodiment, arrangement of the first slit patterns 15a, 15b and the second slit pattern 16a, 16b is not limited thereto. That is, the first slit patterns 15a, 15b and the second slit patterns 16a, 16b do not always need to be arranged line-symmetrically with respect to the axis as long as well-balanced torque transmissibility can be easily ensured in the circumferential cross section.

While the girders 13 for dividing the first slit patterns 15a, 15b as well as the girders 14 for dividing the second slit patterns 16a, 16b are linearly linked along the axis from one end toward the other end of the slitted pipe 10 in the first embodiment, arrangement of the girders 13 and the girders 14 is not limited thereto. That is, the girders 13 as well as the girders 14 do not always need to be linearly linked as long as high transmissibility of push-in torque of the slitted pipe 10 can be ensured. For example, the girders 13 and the girders 14 may be arranged in a staggered manner.

### Second Embodiment

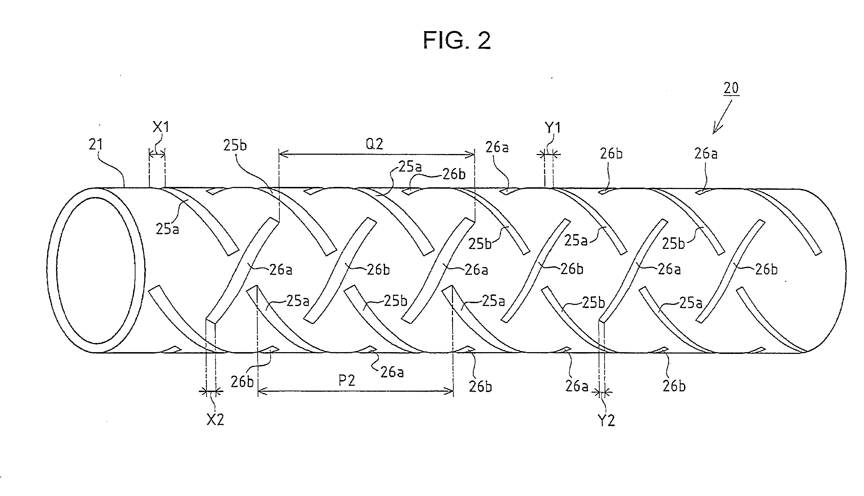

Fig. 2 is a perspective view of a slitted pipe according to a second embodiment of the present invention. As illustrated in Fig. 2, a slitted pipe 20 according to the second embodiment includes a tubular member 21. In the second embodiment, the slit width of first slit patterns 25a, 25b increases from one end (a right side in Fig. 2) toward the other end (a left side in Fig. 2). In addition, the slit width of second slit patterns 26a, 26b also increases from one end (right side) toward the other end (left side).

That is, a slit width X1 of the first slit pattern 25a disposed at the other end (left side in Fig. 2) is larger than a slit width Y1 of the first slit pattern 25a disposed at one end (right side in Fig. 2). In addition, a slit width X2 of the second slit pattern 26a disposed at the other end (left side) of the slitted pipe 20 is larger than a slit width Y2 of the second slit pattern 26a disposed at one end (right side).

According to this, advantages similar to those of the above-described first embodiment can be obtained. Further, high flexibility can be ensured at the other end (left side) of the slitted pipe 20. For example, in a guide wire including the slitted pipe 20, operability is enhanced, and insertability in a blood vessel that intricately branches in a three-dimensional space (vascular selectivity) can be enhanced. In addition, when such a guide wire is inserted in a blood vessel of a patient, perforation of the blood vessel can be prevented effectively.

In the second embodiment, a pitch P2 of the first slit pattern 25a is fixed. Also, a pitch Q2 of the second slit pattern 26a is fixed. The pitch P2 of the first slit pattern 25a and the pitch Q2 of the second slit pattern 26a are equal to each other. Hence, it is possible to enhance torque transmissibility of the slitted pipe 20 itself in a circumferential cross section in a well-balanced manner.

In the second embodiment, as illustrated in Fig. 2, the slit width of the first slit patterns 25a, 25b and the slit width of the second slit patterns 26a, 26b each increase in two steps from one end (right side in Fig. 2) toward the other end (left side in Fig. 2) of the slitted pipe 20. However, the slit width of the first slit patterns 25a, 25b and the slit width of the second slit patterns 26a, 26b may each increase in three or more steps. In this case, advantages similar to those of the second embodiment can also be obtained.

### Third Embodiment

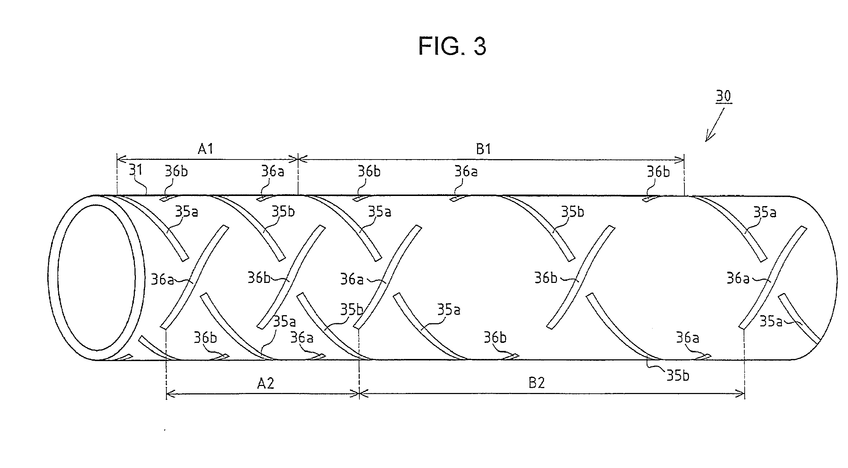

Fig. 3 is a perspective view of a slitted pipe according to a third embodiment of the present invention. As illustrated in Fig. 3, a slitted pipe 30 of the third embodiment includes a tubular member 31. In the slitted pipe 30 of the third embodiment, the pitch of first slit patterns 35a, 35b decreases from one end (a right side in Fig. 3) toward the other end (a left side in Fig. 3). In addition, the pitch of second slit patterns 36a, 36b decreases from one end (right side) toward the other end (left side).

That is, a pitch A1 of the first slit pattern 35a disposed at the other end (left side) of the slitted pipe 30 is less than a pitch B1 of the first slit pattern 35a disposed at one end (right side). In addition, a pitch A2 of the second slit pattern 36a disposed at the other end (left side) of the slitted pipe 30 is less than a pitch B2 of the second slit pattern 36a disposed at one end (right side) .

According to this, advantages similar to those of the above-described first embodiment can be obtained. Further, high flexibility can be ensured at the other end (left side) of the slitted pipe 30. For example, in a guide wire including the slitted pipe 30, operability is enhanced, and insertability in a blood vessel that intricately branches in a three-dimensional space (vascular selectivity) can be enhanced. In addition, when such a guide wire is inserted in a blood vessel of a patient, perforation of the blood vessel can be prevented effectively.

In the third embodiment, preferably, the slit width of the first slit patterns 35a, 36b and the slit width of the second slit patterns 36a, 36b are substantially equal to each other. This can enhance torque transmissibility of the slitted pipe 30 itself in a well-balanced manner in a circumferential cross section.

In the third embodiment, as illustrated in Fig. 3, the pitch of the first slit patterns 35a, 35b and the pitch of the second slit pattern 36a, 36b each decrease in two steps from one end (right side in Fig. 3) toward the other end (left side in Fig. 3). However, the pitch of the first slit patterns 35a, 35b and the pitch of the second slit patterns 36a, 36b may each increase in three or more steps. In this case, advantages similar to those of the third embodiment can also be obtained.

### Fourth Embodiment

Fig. 4 is a perspective view of a slitted pipe according to a fourth embodiment of the present invention. As illustrated in Fig. 4, a slitted pipe 40 of the fourth embodiment includes a tubular member 41. In the slitted pipe 40 of the fourth embodiment, girders 43 are provided to divide first slit patterns 45a, 45b into a plurality of slit groups45a', 45b' each composed of three slits 45a" 45b" disposed adjacent and parallel to one another. Further, second slit patterns 46a, 46b each comprise a plurality of slit groups 46a', 46b' each composed of three slits 46a", 46b" disposed adjacent and parallel to one another is provided to cross the girders 43. The slits 46a", 46b" of the second slit patterns 46a, 46b are not in contact with the slits 45a", 45b" of the first slit patterns 45a, 45b, and the second slit patterns 46a, 46b are disposed at an angle to the first slit patterns 45a, 46b.

In the fourth embodiment, a pitch C1 of the first slit pattern 45a may decrease from one end (a right side in Fig. 4) toward the other end (a left side in Fig. 4), or may be substantially fixed. However, the pitch C1 of the first slit pattern 45a preferably decreases from one end (right side) toward the other end (left side) in order to ensure high flexibility at the other end of the slitted pipe 40. This also applies to the second slit patterns 46a, 46b.

In the fourth embodiment, the slit width of the first slit patterns 45a, 45b may increase from one end (right side in Fig. 4) toward the other end (left side in Fig. 4), or may be substantially fixed. However, the slit width of the first slit patterns 45a, 45b preferably increases from one end (right side) toward the other end (left side) in order to ensure high flexibility at the other end of the slitted pipe 40. This also applies to the second slit patterns 46a, 46b.

The slit number of the slit groups in the first slit patterns 45a, 45b and the slit number of the slit groups of the second slit patterns 46a, 46b are not limited to three, and may be two, or four or more.

### Fifth Embodiment

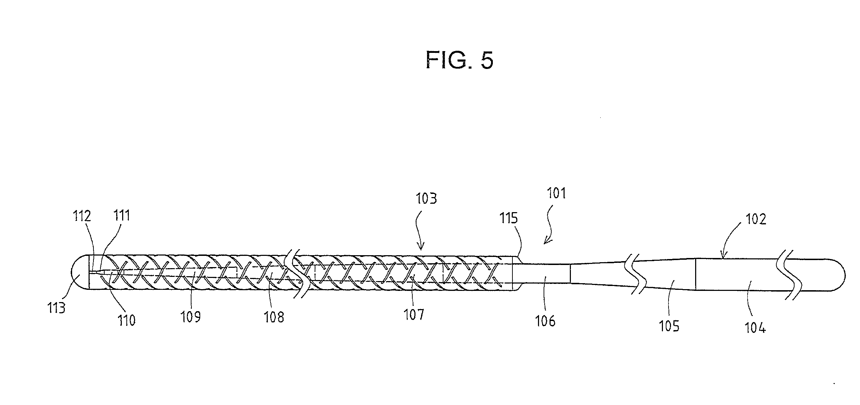

Fig. 5 is an overall view of a first embodiment of a guide wire according to the present invention. In Fig. 5, a left side is a distal side to be inserted into the body, and a right side is a proximal side to be operated by a manipulator such as a doctor. In Fig. 5, the guide wire is schematically illustrated, and the dimensional ratio is different from an actual one.

A guide wire 101 illustrated in Fig. 5 is used, for example, for medical treatment of a blood vessel of the heart. In the fifth embodiment, the guide wire 101 is about 1900 mm in length. The guide wire 101 includes a core shaft 102, a slitted pipe 103 according to any of the above-described first to fourth embodiments, a distal-end brazing part 113 that joins a distal end of the core shaft 102 and a distal end of the slitted pipe 103, and a proximal-end brazing part 115 that joins the core shaft 102 and a proximal end of the slitted pipe 103.

The core shaft 102 includes a first distal end portion 112 provided at the most distal end, a second distal end portion 111 adjacent to a proximal end of the first distal end portion 112, a third distal end portion 110 adjacent to a proximal end of the second distal end portion 111, a first tapered portion 109 adjacent to a proximal end of the third distal end portion 110, a second tapered portion 108 adjacent to a proximal end of the first tapered portion 109, a third tapered portion 107 adjacent to a proximal end of the second tapered portion 108, a first cylindrical portion 106 adjacent to a proximal end of the third tapered portion 107, a fourth tapered portion 105 adjacent to a proximal end of the first cylindrical portion 106, and a cylindrical proximal end portion 104 adjacent to a proximal end of the fourth tapered portion 105.

While the material of the core shaft 102 is not particularly limited, for example, stainless steel (SUS304), a superelastic alloy such as a Ni-Ti alloy, or a piano wire can be used.

An outer surface of a section extending from a distal end of the guide wire 101 to the fourth tapered portion 105 of the core shaft 102 via the slitted pipe 103 is coated with a hydrophilic coating agent. Examples of hydrophilic coating agents are a cellulosic high polymer, a polyethylene oxide high polymer, a maleic anhydride high polymer (for example, a maleic anhydride copolymer such as a methyl vinyl ether-maleic anhydride copolymer), an acrylamide high polymer (for example, a block copolymer of polyacrylamide and poly(glycidyl methacrylate-dimethylacrylamide), hydrosoluble nylon, polyvinyl alcohol, polyvinylpyrrolidone, and hyaluronate.

Since the guide wire 101 of the fifth embodiment includes the slitted pipe 103 covering a distal end portion of the core shaft 102, when the distal end portion of the guide wire 101 is pushed into the blood vessel while the proximal end portion of the guide wire 101 is being rotated in both clockwise and counterclockwise directions, as described above, equivalently high transmissibility of push-in torque can be obtained in both the clockwise and counterclockwise directions. Therefore, it is considerably easy to advance the guide wire 101 into, for example, a chronic total occlusion lesion. This enhances operability.

In the second and third embodiments, the other end of the slitted pipe is preferably located on a distal side of the core shaft 102. In this case, flexibility at the distal end portion of the guide wire 101 can be further enhanced by the slitted pipe.

A gap is preferably formed between the core shaft 102 and the slitted pipe 103. In this case, flexibility can be enhanced when the guide wire 101 is curved, and the guide wire 101 can be easily inserted to an end portion of the blood vessel.

### Sixth Embodiment

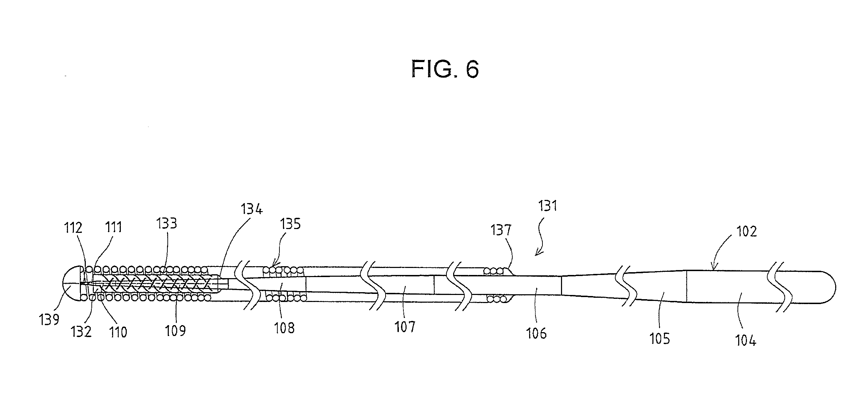

Fig. 6 is an overall view of a second embodiment of a guide wire according to the present invention. In Fig. 6, a left side is a distal side to be inserted into the body, and a right side is a proximal side to be operated by a manipulator such as a doctor. In Fig. 6, the guide wire is schematically illustrated, and the dimensional ratio is different from an actual one.

As illustrated in Fig. 6, a guide wire 131 of the sixth embodiment includes a core shaft 102, an outer coil 135 covering a distal end portion of the core shaft 102, a slitted pipe 133 according to any of the first to fourth embodiments, which is disposed in the outer coil 135 to cover the distal end portion of the core shaft 102, a distal-end brazing part 139 that joins a distal end of the core shaft 102 and a distal end of the outer coil 135, an inner distal-end brazing part 132 that joins a distal end of the slitted pipe 133 and the core shaft 102, an inner proximal-end brazing part 134 that joins a proximal end of the slitted pipe 133 and the core shaft 102, and a proximal-end brazing part 137 that joins the core shaft 102 and a proximal end of the outer coil 135. In the sixth embodiment, advantages similar to those of the above-described fifth embodiment can be obtained.

In the guide wire 131 of the sixth embodiment, the distal end of the slitted pipe 133 is located a predetermined distance apart from the distal end of the core shaft 102 toward the proximal end. In this case, flexibility at the distal end portion of the guide wire 131 can be enhanced further. Hence, the distal end portion of the guide wire 131 can be easily bent, and vascular selectivity of the guide wire 131 can be improved.

Gaps are preferably provided between the core shaft 102 and the slitted pipe 133 and between the outer coil 135 and the slitted pipe 133. In this case, flexibility can be further enhanced when the guide wire 131 is curved, and the guide wire 131 can be easily inserted to an end portion of the blood vessel.

While the distal end of the slitted pipe 133 is located a predetermined distance apart from the distal end of the core shaft 102 toward the proximal end in the sixth embodiment, the distal end of the slitted pipe 133 and the distal end of the core shaft 102 do not always need to be apart from each other as long as flexibility of the distal end portion of the guide wire 131 can be ensured. That is, the distal end of the slitted pipe 133 may be joined to the distal end of the core shaft 102 by the distal-end brazing part 139.

## Claims

1. A slitted pipe comprising:
at least one first slit pattern (15a, 25a, 35a, 45a) helically extending in a longitudinal direction of the slitted pipe and divided by girders (13) at predetermined intervals; and
at least one second slit pattern (16a, 26a, 36a, 46a) helically extending in the longitudinal direction and disposed at an angle to the first slit pattern (15) to cross the girders (13).

2. The slitted pipe according to Claim 1, wherein the first slit pattern (15a, 25a, 35a, 45a) and the second slit pattern (16a, 26a, 36a, 46a) are arranged line-symmetrically with respect to an axis.

3. The slitted pipe according to Claim 1 or 2, wherein the second slit pattern (16a, 26a, 36a, 46a) extending in the longitudinal direction to be helical in a direction opposite from a helical direction of the first slit pattern (15a, 25a, 35a, 45a).

4. The slitted pipe according to Claim 1 or 3, wherein slits of the first slit pattern (15a, 25a, 35a, 45a) are not in contact with slits of the second slit pattern (16a, 26a, 36a, 46a).

5. The slitted pipe according to any of Claims 1 to4 comprising girders (13) which are linearly linked along an axis from one end toward the other end.

6. The slitted pipe according to any of Claims 1 to 5, wherein a slit width of the first slit pattern (25a) and a slit width of the second slit pattern (26a) gradually increase from one end toward the other end.

7. The slitted pipe according to any of Claims 1 to 6, wherein a pitch of the first slit pattern (35a) and a pitch of the second slit pattern (36a) gradually decrease from one end toward the other end.

8. The slitted pipe according to any of Claims 1 to 7, wherein the first slit pattern (45a) and/or the second slit pattern (46a) is/are divided by the girders (13) into a plurality of slit groups (45a' , 46b') composed of a plurality of slits (45a'' , 46b'' ) disposed adjacent and parallel to one another.

9. The slitted pipe according to any of Claims 1 to 8, wherein the slitted pipe is shaped like a substantially perfect circle in cross section.

10. A guide wire comprising:
a core shaft (102); and
the slitted pipe according to any of Claims 1 to 9, the slitted pipe covering a distal end portion of the core shaft (102).

11. The guide wire according to Claim 10 further comprising:
a coil body (135) covering a distal end portion of the core shaft (102), wherein the slitted pipe is disposed in the coil body (135) to cover the distal end portion of the core shaft (102).
